# EUROPÄISCHE PATENTSCHRIFT

(11) **EP 0 531 845 B1**
(45) Veröffentlichungstag und Bekanntmachung des Hinweises auf die Patenterteilung: **07.08.1996**
(21) Anmeldenummer: 92114835.9
(22) Anmeldetag: 31.08.1992
(51) Int. Cl.: A61K 31/44, C07D 211/90

(54) **Pharmazeutische Zubereitungen mit einer speziellen Kristallmodifikation des 1,4-Dihydro-2,6-dimethyl-4-(3-nitrophenyl)-3,5-pyridindicarbonsäure-isopropyl-(2-methoxyethyl)-esters**
Pharmaceutical composition comprising a special crystall modification of 1,4-dihydro-2,6-dimethyl-4-(3-nitrophenyl)-3,5-pyridinedicarbonylic acid-(-2-methoxyethyl)-isopropyl ester
Composition pharmaceutique comprenant une modification crystalline spéciale de la 1,4-dihydro-2,6-diméthyl-4-(3-nitrophényl)-3,5-pyridine dicarboxylic acide-(-2-méthoxyéthyl)-isopropyl ester

(30) Priorität: 11.09.1991 DE 4130173
(43) Veröffentlichungstag der Anmeldung: 17.03.1993
(73) Patentinhaber: BAYER AG, 51368 Leverkusen (DE)
(72) Erfinder: Grunenberg, Alfons, Dr., W-4047 Dormagen (DE); Hegasy, Ahmed, Dr., W-5090 Leverkusen (DE); Mück, Wolfgang, Dr., W-5600 Wuppertal (DE); Franckowiak, Gerhard, Dr., W-5600 Wuppertal (DE); Kanikanti, Rango-Rao, Dr., W-5090 Leverkusen (DE)

(56) Entgegenhaltungen:
- EP-A- 0 004 650
- EP-A- 0 167 909
- GB-A- 2 211 188
- ACTA CRYST. Bd. C45, 1989, Seiten 1748 - 1751 S.D. WANG ET AL. 'STRUCTURE OF THE CALCIUM CHANNEL ANTAGONIST, NIMODIPINE'
- JOURNAL OF COMPUTER-AIDED MOLECULAR DESIGN Bd. 4, Nr. 3, 1990, Seiten 215 - 230 D.A. LANGS ET AL. 'RECEPTOR MODEL FOR THE MOLECULAR BASIS OF TISSUE SELECTIVITY OF 1,4-DIHYDROPYRIDINE CALCIUM CHANNEL DRUGS'
- 'THE MERCK INDEX' 1989 , MERCK & CO., INC. , RAHWAY, N.J., U.S.A. Seite 1036

## Beschreibung

Die Erfindung betrifft pharmazeutische Zubereitungen, enthaltend eine spezielle Kristallmodifikation (nachfolgend "Modifikation II" genannt) des 1,4-Dihydro-2,6-dimethyl-4-(3-nitrophenyl)-3,5-pyridindicarbonsäureisopropyl-(2-methoxyethyl)-esters (Nimodipin) und ein Verfahren zu ihrer Herstellung.

Nimodipin, seine Herstellung und seine Verwendung als Zerebraltherapeutikum sind bereits bekannt (vgl. EP-B-4650 und GB 1 358 951). Die bisher in pharmazeutischen Zubereitungen eingesetzten Nimodipin-Kristalle (Modifikation I) haben einen Schmelzpunkt von 125°C (vgl. The Merck Index (1989) 1036). Von Wang et al. [S.D. Wang, L.G. Herbette, D. G. Rhodes, Acta Cryst. C 45, 1748 (1989)] wurde die Röntgenstruktur eines Nimodipin Enantiomeren (Schmelzbereich 134 - 136°C) beschrieben, die mit der Röntgenstruktur der Modifikation II der Racemform identisch ist.

Die Kristallmodifikation I mit einem Schmelzpunkt von 125°C, die bisher ausschließlich für die Herstellung von Arzneiformen verwendet wurde, kann bei der Herstellung verschiedener Arzneiformen nicht befriedigen. So wird beispielsweise bei Suspensionen für die orale Applikation, die Nimodipin in der Modifikation I enthalten, unter Lagerungsbedingungen Kristallwachstum und in der Folge eine verstärkte Sedimentation beobachtet, die zu Feststoffablagerungen am Boden des Vorratsgefäßes führt, wodurch die Qualität und Dosiergenauigkeit und auch die biologische Wirkung dieser Präparate erheblich beeinträchtigt werden.

Die physikalische Instabilität der bisher bekannten Nimodipin Modifikation I in wäßrigen Suspensionen, die bevorzugt auftritt, wenn die Präparate einer Temperaturbelastung ausgesetzt oder über einen längeren Zeitraum gelagert werden, beeinträchtigt die Resorption, Wirksamkeit und Sicherheit dieser Präparate. Es ist deshalb von großer Bedeutung, eine möglichst stabile Suspension für die Herstellung von Nimodipin enthaltenden Arzneiformen zu verwenden.

Die Nimodipin Modifikation II ist weiß und gegenüber der bekannten gelben Modifikation I überraschenderweise weniger lichtempfindlich und chemisch und physikalisch stabiler. Sie besitzt einen Schmelzpunkt von ca. 116°C. Sie ist für die Herstellung von stabilen und lagerfähigen pharmazeutischen Präparaten die Nimodipinkristalle enthalten, insbesondere für Suspensionen sehr gut geeignet.

Gegenstand der Erfindung sind demnach stabile Arzneizubereitungen, die die kristalline Modifikation II des 1,4-Dihydro-2,6-dimethyl-4-(3-nitrophenyl)-3,5-pyridindicarbonsäure-isopropyl-(2-methoxyethyl)-esters (Nimodipin) mit dem Schmelzpunkt ca. 116°C mit einer mittleren Teilchengröße von höchstens 5 µm enthalten, insbesondere solche Zubereitungen, in denen Kristalle mit einer mittleren Teilchengröße von 0,1 bis 5,0 µm, vorzugsweise 0,3 bis 3,0 µm erforderlich sind wie z.B. Suspensionen oder feste Arzneiformen.

Die Herstellung der erfindungsgemäß zu verwendenden Kristalle der Modifikation II erfolgt z.B., indem man Nimodipin der üblichen Modifikation I in inerten organischen Lösemitteln z.B. in Aceton oder niederen Alkoholen mit vorzugsweise 1 bis 6 C-Atomen gegebenenfalls in Gegenwart von Wasser (Suspensionsflüssigkeit) suspendiert und bis zur quantitativen Umwandlung in die Modifikation II bei Temperaturen von 0 bis 80°C, vorzugsweise 10 bis 60°C, insbesondere 20 bis 50°C behandelt, und gegebenenfalls die erhaltenen Kristalle der Modifikation II nach üblichen Methoden abtrennt und zur Entfernung vorhandenen Lösemittels bei Temperaturen zwischen 20 und 70°C bis zur Gewichtskonstanz trocknet.

Die Umwandlungsdauer beträgt in der Regel ca. 2 bis 24 Stunden und ist abhangig von der Art des Lösemittels und der gewählten Temperatur.

Zur Herstellung von Arzneiformen wird Nimodipin Modifikation II in geeigneter Suspensionsflüssigkeit suspendiert, gegebenenfalls unter Zugabe von pharmazeutischen Hilfsstoffen und die Suspension gemahlen bis zu der gewünschten erfindungsgemäßen maximalen mittleren Teilchengröße, z.B. durch Perlmahlung.

Die Kristallmodifikation II besitzt ein charakteristisches IR-Spektrum, das sich deutlich von dem Spektrum der Modifikation I unterscheidet.

Auch die Röntgenstrukturanalysen zeigen, daß die Molekülkonformation und Anordnung der Moleküle im Kristallgitter von Modifikationen I und II unterschiedlich sind.

Der Schmelzpunkt der Modifikation II liegt bei 116°C und ist somit klar unterscheidbar gegenüber 125°C (in einigen Publikationen sind Bereiche von 123 bis 127°C angegeben) von Modifikation I. Die reinen Enantiomere des Nimodipins schmelzen bei 134 - 136°C.

Das mittels DSC (Differential Scanning Calorimetry) unter Atmosphärendruck aufgenommene Thermogramm der Modifikation II zeigt in Übereinstimmung mit der Schmelzpunktsbestimmung einen endothermen Schmelzpeak bei 116°C und unterscheidet sich eindeutig vom Thermogramm der Modifikation I.

Die Röntgendiffraktogramme und ¹³C-Festkörper-NMR-Spektren sind charakteristisch unterschiedlich für die Nimodipin Kristallmodifikationen I und II.

Bisher sind nur Nimodipinzubereitungen im Handel, die den Wirkstoff in amorpher oder gelöster Form enthalten. Nimodipin wird einmal als Tropfen oder Lösung in Kapseln, d.h. gelöst in einer Mischung bestimmter organischer Lösemittel, und als Copräzipitat ("feste Lösung") in festem Polyvinylpyrrolidon in Tablettenform angeboten. Die Herstellung der Copräzipitat-Tablette ist mit hohem technischen Aufwand verbunden und die flüssigen Nimodipin Lösungen zeigen eine sehr schnelle Anflutung der Plasmaspiegel, was häufig zu unerwünschten Wirkungen führt. Aufgabe der Erfindung ist somit das zur Verfügung stellen einer Zubereitungsform für den extrem schwerlöslichen und problematischen Wirkstoff Nimodipin, die
a) in einfacher Weise herstellbar ist,
b) eine gute Bioverfügbarkeit besitzt,
c) eine moderate Anflutung der Plasmaspiegel gewährleistet,
d) leicht dosierbar und applizierbar ist und
e) eine gute Lagerstabilität hat.

Die erfindungsgemäße Kristallmodifikation II des Nimodipin zeigt in pharmazeutischen Präparaten z.B. bei Lagerung im Vergleich zur Modifikation I eine höhere physikalische und chemische Stabilität und ist daher sicherer in der Anwendung und somit für die Herstellung verschiedener Arzneiformen, insbesondere Suspensionen, besser geeignet.

Gegenstand der Erfindung sind auch oral applizierbare flüssige und feste pharmazeutische Zubereitungen, die die Kristallmodifikation II enthalten, wie z.B. Suspensionen, Tabletten, Dragees, Hart- und Weichgelatinekapseln und dergleichen. Von besonderem Nutzen ist die Verwendung in Suspensionen.

Bei Einsatz mehrerer, verschiedener pharmazeutischer Zubereitungen einer Wirksubstanz ist die Bioäquivalenz der Arzneiformulierungen, d.h. die biometrisch abgesicherte Gleichheit von systemisch verfügbarer Dosis und maximaler Plasmakonzentration, von großer Bedeutung für die therapeutische Äquivalenz der pharmazeutischen Zubereitungen.

Mit der im Ausführungsbeispiel 1 angeführten Suspension mit perl-gemahlenem Nimodipin/Kristallmodifikation II ist eine oral einsetzbare Arzneiformulierung verfügbar, die der Nimodipin-Handelstablette (vgl. EP-Patent-Nr. 167909-B) hinsichtlich ihrer pharmakokinetischen Eigenschaften äquivalent ist; d.h. neben der einfachen Herstellbarkeit (a) und der guten Lagerstabilität (e) auch die Bedingungen b), c) und d) erfüllt.

**Tabelle***

| | Kristallsuspension (Nimodipin/Modifikation II) | Handelstablette |
|---|---|---|
| relative Bioverfügbarkeit [%] | 94.2/1.40 | 100 |
| maximale Plasmakonzentration [µg/l] | 11.0/1.71 | 11.3/1.55 |

| | | |
|---|---|---|
| (* Zahlenangaben: geometrischer Mittelwert/geom. Standardabweichung | | |

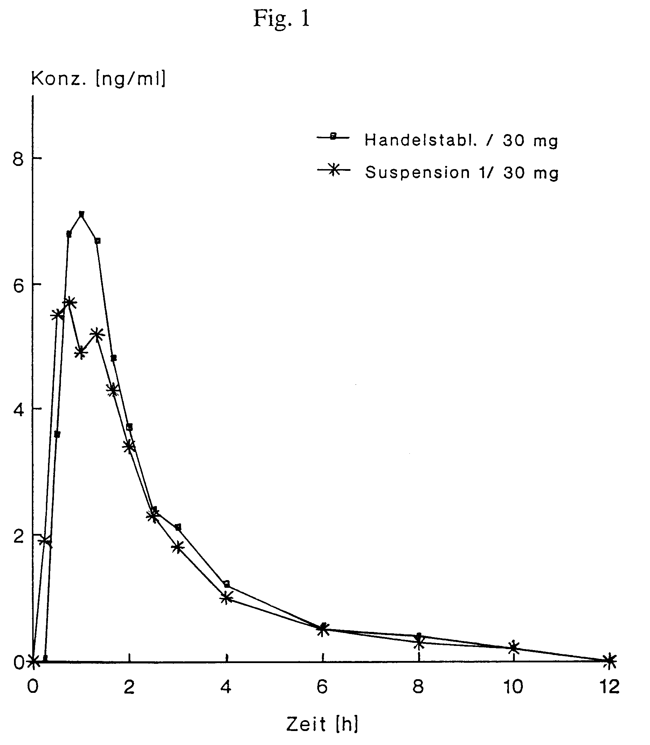

Fig. 1 zeigt die Nimodipin Plasmakonzentrationen (geometrische Mittelwerte) der Handelstablette/30 mg und der erfindungsgemäßen Suspension/30 mg.

Die erfindungsgemäßen pharmazeutischen Zubereitungen enthalten neben Nimodipin der Modifikation II gegebenenfalls einen oder mehrere andere Wirkstoffe als solche oder sind gegebenenfalls zusammen mit in der Galenik üblicherweise eingesetzten Hilfs- und Zusatzmitteln, wie Bindern, Füllstoffen, Konservierungsmitteln, Sprengmitteln, Fließregulierungsmitteln, Weichmachern, Netzmitteln, Dispergiermitteln, Emulgatoren, Lösungsmitteln, Geschmackstoffen und dergleichen zu üblichen Darreichungsformen für die orale oder parenterale Applikation formuliert.

Die Herstellung pharmazeutischer Zubereitungen erfolgt in an sich bekannter Weise, beispielsweise durch Mischen, Einrühren, Suspendieren, Emulgieren usw. der Wirkstoffe mit bzw. in den pharmazeutischen Hilfsstoffen und Verarbeitung zu pharmazeutisch geeigneten Darreichungsformen für die orale, parenterale oder rektale Applikation.

Es war nicht vorhersehbar, daß für den seit vielen Jahren bekannten Wirkstoff Nimodipin eine bisher nicht in Arzneizubereitungen eingesetzte stabile Kristallmodifikation existiert, die bei gleicher biologischer Wirkung eine geringere Lichtempfindlichkeit und eine bessere Lagerstabilität besitzt. Insbesondere bei Suspensionen wird durch die Verwendung der Modifikation II unerwünschtes Kristallwachstum und die Bildung anderer Kristallformen vermieden. Durch den Einsatz der Modifikation II wird die Sicherheit für Nimodipin-Zubereitungen erhöht und somit das Risiko für den Patienten verringert.

Die folgenden Ausführungsbeispiele erläutern den Gegenstand der Erfindung.

### Ausführungsbeispiele

### Beispiel 1

3 g Hydroxypropyl-Cellulose L fine und 0,5 g Solbrol M werden in ca. 450 ml entmineralisiertem Wasser gelöst, und in dieser Lösung werden 30 g mikrofein gemahlenes Nimodipin der Modifikation II suspendiert. Daneben werden 12 g Hydroxypropyl-Cellulose M fine, 1 g Citronensäure, 0,5 g Solbrol M und 2 g tert.-Natriumcitrat in ca. 500 ml entmineralisiertem Wasser gelöst. Die Nimodipin-enthaltende Suspension wird perl-gemahlen und anschließend mit der Suspensionsflüssigkeit bei Raumtemperatur vermischt. Die mittlere Teilchengröße liegt bei 0,9 µm.

### Beispiel 1a (Negativ-Beispiel)

Analog Beispiel 1 werden 30 g Nimodipin der Modifikation I suspendiert und mittels einer Perlmühle gemahlen.

Nach Verdünnung mit der restlichen Suspensionsflüssigkeit werden die Suspensionen in Flaschen abgefüllt und bei ca. 40°C gelagert. Bereits nach 3 Wochen besteht Nimodipin aus Kristallen von einer mittleren Teilchengröße von ca. 20 µm.

### Beispiel 2

Die Suspension, bestehend aus 2 g Arlacel 20, 6 g Tylopur C 300 P, 1 g Citronensäure, 2 g tert.-Natriumcitrat, 0,5 g Solbrol M, 30 g mikrofein gemahlenes Nimodipin der Modifikation II in ca. 500 ml entmineralisiertem Wasser wird einer Perlmahlung unterzogen und anschließend unter Rühren bei Raumtemperatur mit der Suspensionsflüssigkeit, bestehend aus ca. 460 ml entmineralisiertem Wasser, 6 g Tylopur C 300 P, und 0,5 g Solbrol M vermischt. (Mittlere Teilchengröße 1,7 µm).

### Beispiel 3: (Suspensionstropfen mit 6 % Nimodipin)

14,4 g Solbrol M werden in 1593,6 g heißem Wasser gelöst. 72 g Hydroxypropylcellulose L (low viscosity) werden darin suspendiert und diese abgekühlt und gerührt bis klar gelöst. 720 g von mikrofeingemahlenem Nimodipin der Modifikation II werden darin suspendiert. Die Suspension (2,400 g) wird mittels einer Perlmühle unter Kühlung zu einer mittleren Teilchengröße von ca. 0,85 µm gemahlen. In separatem Gefäß werden 8 g Solbrol M, 10 g Citronensäure, 20 g Natriumcitrat und 122 g in 8012 g Wasser entmineralisiert gelöst. Zu dieser Lösung gibt man bei Raumtemperatur 2000 g der gemahlenen Suspension dazu und rührt. Es entstehen Tropfen, die nach Lagerung über 1 Jahr keine Änderung der physikalischen Eigenschaft oder Teilchengröße zeigen.

### Beispiel 4: (Feste Arzneiform)

24 g Hydroxypropylcellulose L und 18 g Texapon K 12 werden in 1040 g Wasser gelöst. 600 g mikronisierte Nimodipin Modifikation II werden darin suspendiert und mittels Perlmühle zu einer mittleren Teilchengröße von ca. 0,3 µm gemahlen. 135 g Hydroxypropylcellulose L werden in 1500 g gelöst. Zu dieser Lösung gibt man 1260 g der perlgemahlenen Nimodipin-Suspension dazu. In einem Wirbelschicht-Granuliergerät werden 450 g Avicel, 561 g Lactose und 300 g AC-Di-Sol vorgelegt, die o.a. Suspension wird gesprüht, die erhaltenen Granulate werden gesiebt und mit 465 g Avicel, 300 g Plasdone XL und 9 g Magnesiumstearat gemischt. Die Mischung wird zu Tabletten mit einem Gesamtgewicht von 270 mg gepreßt und enthalten pro Tablette 45 mg Nimodipin Modifikation II. Die Tabletten werden lackiert.

## Patentansprüche (Patentansprüche für folgende(n) Vertragsstaat(en): AT, BE, CH, DE, DK, FR, GB, IE, IT, LI, LU, MC, NL, PT, SE)

1. Stabile pharmazeutische Zubereitung, enthaltend die spezielle Modifikation II des kristallinen 1,4-Dihydro-2,6-dimethyl-4-(3-nitrophenyl)-3,5-pyridindicarbonsäure-isopropyl-(2-methoxyethyl)-esters mit dem Schmelzpunkt 116°C mit einer mittleren Teilchengröße von höchstens 5 µm.

2. Arzneimittel gemäß Anspruch 1 in Form von Suspensionen, Tabletten oder Kapseln.

3. Verfahren zur Herstellung von Arzneimitteln gemäß Anspruch 1, dadurch gekennzeichnet, daß man kristallines Nimodipin der Modifikation II mit einer mittleren Teilchengröße von höchstens 5 µm gegebenenfalls unter Verwendung von üblichen Hilfs- und Trägerstoffen in eine geeignete Applikationsform überführt.

4. Verfahren zur Herstellung von kristallinem Nimodipin der Modifikation II, dadurch gekennzeichnet, daß man Nimodipin der Modifikation I in inerten organischen Lösemitteln gegebenenfalls in Gegenwart von Wasser suspendiert und bei Temperaturen von 0 bis 80°C bis zur quantitativen Umwandlung in die Modifikation II behandelt und anschließend gegebenenfalls nach Abtrennung und Trocknung zu Kristallen mit einer maximalen mittleren Teilchengröße von 5 µm mahlt.

5. Verwendung von kristallinem Nimodipin der Modifikation II mit einer mittleren Teilchengröße von höchstens 5 µm zur Herstellung von cerebral wirksamen Arzneimitteln.

6. Verwendung gemäß Anspruch 5 zur Herstellung von cerebral wirksamen Suspensionen.

## Patentansprüche (Patentansprüche für folgende(n) Vertragsstaat(en): ES, GR)

1. Verfahren zur Herstellung von stabilen pharmazeutischen Zubereitungen enthaltend die spezielle Modifikation II des kristallinen 1,4-Dihydro-2,6-dimethyl-4-(3-nitrophenyl)-3,5-pyridin-dicarbonsäure-isopropyl-(2-methoxyethyl)-esters mit dem Schmelzpunkt 116°C mit einer mittleren Teilchengröße von höchstens 5 µm, dadurch gekennzeichnet, daß man den kristallinen Wirkstoff der Modifikation II gegebenenfalls unter Verwendung von üblichen Hilfs- und Trägerstoffen in eine geeignete Applikationsform überführt.

2. Verfahren zur Herstellung von kristallinem Nimodipin der Modifikation II, dadurch gekennzeichnet, daß man Nimodipin der Modifikation I in inerten organischen Lösemitteln gegebenenfalls in Gegenwart von Wasser suspendiert und bei Temperaturen von 0 bis 80°C bis zur quantitativen Umwandlung in die Modifikation II behandelt und anschließend gegebenenfalls nach Abtrennung und Trocknung zu Kristallen mit einer maximalen mittleren Teilchengröße von 5 µm mahlt.

3. Verwendung von kristallinem Nimodipin der Modifikation II mit einer mittleren Teilchengröße von höchsten 5 µm zur Herstellung von cerebral wirksamen Arzneimitteln.

4. Verwendung von kristallinem Nimodipin der Modifikation II gemäß Anspruch 3 zur Herstellung von cerebral wirksamen Suspensionen.

## Claims (Claims for the following Contracting State(s): AT, BE, CH, DE, DK, FR, GB, IE, IT, LI, LU, MC, NL, PT, SE)

1. Stable pharmaceutical preparation, containing the specific modification II of crystalline isopropyl-(2-methoxyethyl) 1,4-dihydro-2,6-dimethyl-4-(3-nitrophenyl)-3,5-pyridinedicarboxylate of melting point 116°C having an average particle size of at most 5 µm.

2. Medicament according to Claim 1 in the form of suspensions, tablets or capsules.

3. Process for the preparation of medicaments according to Claim 1, characterised in that crystalline nimodipine of modification II having an average particle size of at most 5 µm is converted into a suitable administration form, if appropriate using customary auxiliaries and excipients.

4. Process for the preparation of crystalline nimodipine of modification II, characterised in that nimodipine of modification I is suspended in inert organic solvents, if appropriate in the presence of water, and treated at temperatures of 0 to 80°C until quantitative conversion to modification II occurs and then ground to give crystals having a maximum average particle size of 5 µm, if appropriate after separation and drying.

5. Use of crystalline nimodipine of modification II having an average particle size of at most 5 µm for the preparation of cerebrally active medicaments.

6. Use according to Claim 5 for the preparation of cerebrally active suspensions.

## Claims (Claims for the following Contracting State(s): ES, GR)

1. Process for the preparation of stable pharmaceutical preparations, containing the specific modification II of crystalline isopropyl-(2-methoxyethyl) 1,4-dihydro-2,6-dimethyl-4-(3-nitrophenyl)-3,5-pyridinedicarboxylate of melting point 116°C having an average particle size of at most 5 µm, characterised in that the crystalline active compound of modification II is converted into a suitable administration form, if appropriate using customary auxiliaries and excipients.

2. Process for the preparation of crystalline nimodipine of modification II, characterised in that nimodipine of modification I is suspended in inert organic solvents, if appropriate in the presence of water, and treated at temperatures of 0 to 80°C until quantitative conversion to modification II occurs and then ground to give crystals having a maximum average particle size of 5 µm, if appropriate after separation and drying.

3. Use of crystalline nimodipine of modification II having an average particle size of at most 5 µm for the preparation of cerebrally active medicaments.

4. Use of crystalline nimodipine of modification II according to Claim 3 for the preparation of cerebrally active suspensions.

## Revendications (Revendications pour l'(les) Etat(s) contractant(s) suivant(s): AT, BE, CH, DE, DK, FR, GB, IE, IT, LI, LU, MC, NL, PT, SE)

1. Préparation pharmaceutique stable contenant la modification spécifique II de l'ester isopropylique, (2-méthoxy)éthylique de l'acide 1,4-dihydro-2,6-diméthyl-4-(3-nitrophényl)-3,5-pyridine-dicarboxylique cristallin dont le point, de fusion est de 116°C et dont la granulométrie moyenne maximale est de 5 µm.

2. Médicament selon la revendication 1, sous la forme de suspensions, de comprimés ou de capsules.

3. Procédé pour la préparation d'un médicament selon la revendication 1, caractérisé en ce qu'on transforme de la nimodipine cristalline de la modification II, dont la granulométrie moyenne maximale est de 5 µm, éventuellement en utilisant des substances auxiliaires et de support habituelles, en une forme d'application appropriée.

4. Procédé pour la préparation de nimodipine cristalline de la modification II, caractérisé en ce qu'on met de la nimodipine de la modification I en suspension dans des solvants organiques inertes éventuellement en présence d'eau et on la traite à des températures de 0 à 80°C jusqu'à ce que l'on obtienne la transformation quantitative en modification II et ensuite, éventuellement, après séparation et séchage, on broie pour obtenir des cristaux possédant une granulométrie moyenne maximale de 5 µm.

5. Utilisation de nimodipine cristalline de la modification II, dont la granulométrie moyenne maximale est de 5 µm, pour la préparation de médicaments à activité cérébrale.

6. Utilisation selon la revendication 5, pour la préparation de suspensions à activité cérébrale.

## Revendications (Revendications pour l'(les) Etat(s) contractant(s) suivant(s): ES, GR)

1. Procédé pour la formulation de préparations pharmaceutiques stables contenant la modification spécifique II de l'ester isopropylique, (2-méthoxy)éthylique de l'acide 1,4-dihydro-2,6-diméthyl-4-(3-nitrophényl)-3,5-pyridine-dicarboxylique cristallin dont le point de fusion est de 116°C et dont la granulométrie moyenne maximale est de 5 µm, caractérisé en ce qu'on transforme la substance active cristalline de la modification II, éventuellement en utilisant des substances auxiliaires et de support habituelle, en une forme d'application appropriée.

2. Procédé pour la préparation de nimodipine cristalline de la modification II, caractérisé en ce qu'on met de la nimodipine de la modification I en suspension dans des solvants organiques inertes éventuellement en présence d'eau et on la traite à des températures de 0 à 80°C jusqu'à ce que l'on obtienne la transformation quantitative en modification II et ensuite, éventuellement, après séparation et séchage, on broie pour obtenir des cristaux possédant une granulométrie moyenne maximale de 5 µm.

3. Utilisation de nimodipine cristalline de la modification II, dont la granulométrie moyenne maximale est de 5 µm, pour la préparation de médicaments à activité cérébrale.

4. Utilisation de nimodipine cristalline de la modification II selon la revendication 3, pour la préparation de suspensions à activité cérébrale.
